Europäisches Patentamt

European Patent Office

Office européen des brevets

(11) Publication number: **0 625 574 A1**

(12) **EUROPEAN PATENT APPLICATION**

(21) Application number: **94107586.3**

(22) Date of filing: **17.05.94**

(51) Int. Cl.⁵: **C12N 15/12**, C12N 15/85,
C12N 15/18, C12P 21/00,
C12N 5/06, C12N 5/16

(30) Priority: **19.05.93 JP 117283/93**
**14.04.94 JP 75553/94**

(43) Date of publication of application:
**23.11.94 Bulletin 94/47**

(84) Designated Contracting States:
**AT BE CH DE DK ES FR GB GR IE IT LI LU NL
PT SE**

(71) Applicant: **Takeda Chemical Industries, Ltd.**
**1-1 Doshomachi 4-chome,**
**Chuo-ku**
**Osaka-shi, Osaka 541 (JP)**

(72) Inventor: **Sawada, Hidekazu**
**531-Oaza-takamiya**
**Neyagawa, Osaka 572 (JP)**
Inventor: **Takekawa, Shiro**
**5-3-B305, Umezono 2-chome**
**Tsukuba, Ibaraki 305 (JP)**
Inventor: **Nishimura, Osamu**
**54-16, Daiwanishi 1-chome**
**Kawanishi, Hyogo 666-01 (JP)**

(74) Representative: **von Kreisler, Alek,**
**Dipl.-Chem. et al**
**Patentanwälte**
**von Kreisler-Selting-Werner**
**Bahnhofsvorplatz 1 (Deichmannhaus)**
**D-50667 Köln (DE)**

(54) **Production for biologically active polypeptide.**

(57) The present invention provides a method of producing a biologically active polypeptide by culturing animal cells capable of producing the polypeptide, adapted to agitation culture in suspension in serum-free medium, and Chinese hamster ovarian cells, capable of proliferation in suspension in serum-free medium, which produce a polypeptide belonging to the human nerve growth factor family and is useful for industrial mass production of a biologically active polypeptide in high purity.

EP 0 625 574 A1

Field of the invention

The present invention relates to a method of efficiently producing a eukaryotic biologically active polypeptide by culturing recombinant animal cells in suspension in serum-free medium, and cells capable of producing the biologically active polypeptide by the culture of the present invention.

Background of the invention

The mass-culture of recombinant animal cells is very important for the production of biologically active polypeptides such as interferons, erythropoietin, tissue plasminogen activator, granulocyte colony stimulating factors and nerve growth factors.

For example, the nerve growth factor (hereinafter sometimes abbreviated to as NGF) family is known to play a key role in the survival of cholinergic neurons in the procephalic basal field in the central nervous system, as well as being essential for the survival of sensory or sympathetic nerve ganglion cells in the peripheral nervous system. Since NGF acts to enhance neuron survivability as stated above; since intracerebral administration of NGF improved memory disorder in aged rats [W. Fischer et al., Nature, Vol. 329, p. 65 (1887)]; and since research has advanced drug delivery systems for intracerebral transportation of intravenously administered NGF [P.M. Friden et al., Science, Vol. 259, p. 373 (1993)], there is a need for industrial production of NGF with the expectation that it is applicable to pharmaceuticals for senile dementia.

For producing biologically active polypeptides by animal cell, both suspensive and adherent types of cells can be used for host cells: suspensive cells such as human Burkitt's lymphoma (Namalwa) cells and mouse myeloma (Sp2/0) cells, and adhesive cells such as Chinese hamster ovary (CHO) cells, baby hamster kidney (BHK) cells, human uterocervical epitheloid carcinoma (HeLa) cells, mouse mammary tumor (C127) cells and mouse fibroblast (NIH/3T3) cells. CHO cells, in particular, are commonly used [C. Bebbington and C. Hentschel, Trends in Biotechnology, Vol. 3, p. 314 (1985); D. Broad et al., Cytotechnology, Vol. 5, p.47 (1991)].

Suspensive cells usually permit agitation culture in suspension, while adhesive cells, not easy to culture in suspension, are usually cultured by roller bottle culture, microcarrier culture or culture in which cells are immobilized on an immobilizing bed. For industrial mass production of valuable substances, however, suspension culture is advantageous over immobilized culture from the viewpoint of operation and scale-up feasibility. Recently it was reported that the erythroblast differentiation factor (EDF) gene introduced adhesive CHO cells were adapted to suspension culture by forcedly repeating culture in suspension [M. Murata et al., Journal of Fermentation Technology, Vol. 66, p. 501 (1988)], but these cells were only viable for five days in serum-free medium.

The above-mentioned host cells all require serum for their proliferation, and are usually incapable of proliferating in serum-free medium. Serum contains various unknown protein substances and its quality fluctuates among lots. Because of these features, serum interferes with purification of the desired biologically active polypeptide. In pharmaceuticals, in particular, the use of serum-containing medium requires painstaking tests concerning removal of serum-derived impurities for safety evaluation. In addition, serum is expensive. Accordingly, the serum-containing medium is variously problematic for industrial production of a substance, and the use of serum-free medium is desired. In recent years, there have been some reports of adaptation to serum-free medium of serum-dependent cells such as Namalwa cells or the antithrombin III gene transfected CHO cells [S. Hosoi et al., Cytotechnology, Vol. 5, p. 17 (1991); T. Yamauchi et al., Biosci. Biotech. Biochem., Vol. 56, p. 600 (1990)]. It is necessary, however, to determine what kinds of medium are suitable to serum-free medium adaptation on a cell-by-cell basis because commonly used serum-free media are diverse.

Also, cell lines adapted to serum-free medium do not always retain their original productivity; a highly productive strain must be bred again in the case of productivity reduction.

For efficient production of substances by cell culture, it is usually advantageous to continuously culture cells at high density for a long period of time. In culturing suspensive cells such as hybridomas for producing monoclonal antibodies and recombinant mouse myeloma cells encoding desired substance, high cell density perfusion culture in suspension has been attempted in which cells are continuously cultured for a long period of time while fresh medium is supplied to the culture vessel and culture supernatant containing waste products is removed from the culture vessel [K. Kitano et al., Applied Microbiology and Biotechnology, Vol. 24, p. 282 (1986); Y. Takazawa and M. Tokashiki, Cytotechnology, Vol. 2, p. 95 (1989)]. However, there are no animal cells adapted to both of suspension culture and serum independence which were originally adhesive and serum-dependent.

2

Although the development of mass production methods of various biologically active polypeptides is desired, as described above, no such production methods have been well established. For production of human NGF of high specific activity by cell culture, for instance, the use of the human NGF gene introduced CHO cells [Iwane et al., Biochemical and Biophysical Research Communication, Vol. 171, p. 116 (1990)] is best. This method, however, uses a medium containing about 5% fetal calf serum, and all proposed culture designs are of small laboratory scale and short term. For industrial mass production of biologically active polypeptides, it is necessary to improve production methods and increase the production scale.

Summary of the invention

The present inventors investigated methods of efficiently producing the desired biologically active polypeptide using recombinant CHO cells that produce human NGF, and discovered a technique for very efficient production of biologically active polypeptides such as the human NGF family. The inventors made further investigations based on this finding, and developed the present invention.

Accordingly, the present invention relates to a method of producing a biologically active polypeptide for extended period of time which comprises adapting adhesion-dependent cells, i.e., adhesive animal cells, capable of producing said biologically active polypeptide, to be adhension-independent, i.e., adapted to agitation culture in suspension and adapting said cells to grow in serum-free medium. Preferably, the extended period of time for production of the biologically active polypeptide is more than 20 days, more preferably, more than 40 days and most preferably, more than 60 days. The animal cells are preferably Chinese hamster ovary cells, and the biologically active polypeptide is preferably a peptide belonging to the human nerve growth factor family, especially NGF or NGF-2, further the culture method is preferably agitation culture in suspension and also perfusion culture. The present invention also relates to Chinese hamster ovary cells which produce a peptide belonging to the human nerve growth factor family and which is capable of proliferation in serum-free medium with suspension for extended period of time, more specifically the polypeptide is NGF or NGF-2.

Brief description of the drawings

Figure 1 shows the time course of serum-free batch culture in suspension of SF-K45 obtained in Example 4.

Figure 2 shows the time course of serum-free perfusion culture in suspension of SF-K45 obtained in Example 4.

Figure 3 shows results of SDS-PAGE of the preparation of the purified human NGF obtained in Example 4. In these figures, M represents a molecular weight marker, A represents the preparation of purified human NGF obtained in Example 4 and B represents the preparation of mouse NGF respectively.

Detail of the invention

Adhesive animal cells for the present invention include the cells capabel of producing biologically active polypeptides such as Chinese hamster ovary (CHO) cells, baby hamster kidney (BHK) cells, human uterocervical epitheloid carcinoma HeLa cells, mouse mammary tumor C127 cells, mouse fibroblast NIH/3T3 cells, BALB3T3 cells, African green monkey kidney Vero cells and Vero cell substrain Verots S3, with preference given to CHO cells.

The biologically active polypeptide in the present invention is exemplified by hormones, analgesic substances, lymphokines, blood cell acting factors, enzymes, nerve transmitters, growth factors and active derivatives or muteins thereof. Specifically, hormones include luteinizing hormone-releasing hormone (LH-RH), thyroid hormone releasing hormone (TRH), insulin, somatostatin, growth hormones, prolactin, adrenocorticotropic hormone (ACTH), melanocyte-stimulating hormone (MSH), thyroid-stimulating hormone (TSH), luteinizing hormone (LH), follicle-stimulating hormone (FSH), vasopressin, and derivatives thereof leg., desmopressin, Folia Endocrinologica Japonica, Vol. 54, No. 5, pp. 676-691 (1978)], oxytocin, calcitonin, parathyroid hormone (PTH) or fragment thereof (e.g. PTH (l-34)), glucagon, gastrin, secretin, pancreozymin, cholecystokinin, angiotensin, human placental lactogen, human chorionic gonadotropin (HCG), caerulein and motilin. Analgesic substances include enkephalin and derivatives thereof (see US Patent No. 4,277,394 and European Patent Publication No. 31567), endorphin, daynorphin and kyotorphin. Lymphokines include interferons ($\alpha$, $\beta$, $\gamma$) and interleukins (IL-1, -2, -3, -4, -5, -6, -7, -8, -9, -10, -11). Blood cell acting factors include granulocyte colony stimulating factor, granulocyte macrophage colony stimulating factor, macrophage colony stimulating factor, erythropoietin, thymopoietin and thymosin. Enzymes include urokinase, tissue plasminogen activator and caerulein. Nerve transmitters include bombesin, neurotensin, bradykinin and substance P. Growth factors include the nerve growth factor (NGF) family, (NGF, BDNF, NGF-2, NT-4 NT-5) epithelial growth factor (EGF) and the fibroblast growth factor (FGF) family (aFGF, bFGF, INT-2 HST-1, FGF-5, FGF-6 etc.).

The present invention is preferably used to produce biologically active polypeptides which are difficult to obtain in large amounts because of their very limited natural abundance, such as growth factors, particularly the peptides belonging to the nerve growth factor family, e.g., NGF and NGF-2 [FEBS Letters, Vol. 266, p. 187 (1990); with the designation NT-3, A. Hohn et al., Nature, Vol. 344, p. 399 (1989)] and BDNF [B. Knusel et al., Proceedings of National Academy of Science, Vol. 88, p. 961 (1991); A. Rosenthol et al., Neuron, Vol. 4, p. 767 (1990)].

Although the present invention is applicable to peptides, whether natural or non-natural biologically active polypeptides such as mutein or active derivative, it can be advantageously used for biologically active polypeptides whose bioactivity is significantly affected by steric structure and sugar chains.

As for adhesive animal cells capable of producing a biologically active polypeptide that are used for the production method of the present invention, already-established strains with such nature may be used. Where necessary, such animal cells may be obtained by introducing the gene encoding the biologically active polypeptide into the adhesive animal cell by a known genetic engineering technique. Using a strain having high productivity of desired polypeptide is preferable.

Specifically, for the present invention, adhesive animal cells which produce a biologically active polypeptide can be obtained by 1) preparing a gene encoding the desired biologically active polypeptide from a human genomic library or human cDNA library by cloning, or from human genomic DNA, mRNA or cDNA by the polymerase chain reaction (PCR) method, or by chemical synthesis, 2) ligating an appropriate promotor (e.g., SV40-derived promotor, retrovirus promotor or cytomegalovirus promoter), promotor-pre-pro or promotor-signal (e.g., IL-2 gene signal) to the gene as necessary, 3) inserting the ligation product to an appropriate vector, and 4) transforming the adhesive animal cells using the thus-obtained vector.

Example animal cell lines thus obtained include those producing a peptide belonging to the NGF family, such as NGF-producing CHO cells which include CHO-D31-10 used in Examples below and CHO-D31-10-2 (EP 0414151), and NGF-2-producing CHO cells which include CHO-N2-37, CHO-dN2-17, CHO-dN2-19 and CHO-N2-1 (EP 0386752).

In the present invention, adaptation of adhesive animal cells to agitation culture in suspension in serum-free medium is usually achieved by a method in which the cells are first adapted to agitation culture in suspension using serum-containing medium (A) and then adapted to serum-free medium (B); however, it is of course possible to use the method in which the serum concentration is gradually lowered to a serum-free state under agitated suspension culture conditions, i.e., the method in which (A) and (B) are simultaneously performed. In another useful method, cells are first adapted to serum-free medium by a static culture method such as plate culture, and then adapted to agitation culture in suspension.

For adaptation to agitation culture in suspension using serum medium (A), a common basal medium for animal cell culture supplemented with about 0.1 to about 10%, preferably about 0.5 to about 5%, fetal calf serum (FCS) is used, and a serum-derived growth factor fraction [GFS; Tsukamoto et al., Proceedings of the 2nd Assembly of the New Generation Industrial Fundamental Technology Symposium - Biotechnology, p. 175 (1984)] is used as a serum substitute. Basal media that can be used include Eagle MEM medium, Dalbecco's modified Eagle medium (DMEM), Iscove's medium [N. Iscove and F. Melchers, Journal of Experimental Methods, Vol. 147, p. 923 (1978)], Ham's F12 medium [R.G. Ham, Proceedings of National Academy of Science, Vol. 53, p. 288 (1965)], L-15 medium [A. Leibovitz, American Journal of Hygiene, Vol. 78, p. 173 (1963)], RPMI 1640 medium [G.E. Moore et al., The Journal of the American Medical Association (J.A.M.A.), Vol. 199, p. 519 (1967)], ASF-104 medium (Ajinomoto Co., Inc.), CHSF medium (Gibco), COSMEDIUM-001 medium (Cosmo Bio), E-RDF medium (Kyokuto Seiyaku), UC-103 medium (Nissui Pharmaceutical), UC-202 medium (Nissui Pharmaceutical), S-Clone SF-02 medium (Sanko Junyaku Co., Ltd.), T medium (Nihon Seiyaku, Japanese Patent Unexamined Publication No. 145088/1985), TL-2 medium [Y. Shintani et al., Applied Microbiology and Biotechnology, Vol., 27, p. 533 (1988)] and appropriate ratio mixtures thereof. Among the above basal media, ASF-104 medium, E-RDF medium TE medium and appropriate ratio mixtures thereof are preferable.

For adaptation to serum-free medium (B), the above-mentioned common basal media may be used after removing of serum and serum substitutes, and adding amino acids, vitamins, nucleic acids, growth factors, hormones and other low molecular compounds singly or in combination at appropriate concentrations. Specifically, media supplemented with insulin, transferrin, ethanolamine, selenium, proline, polyethylene glycol (PEG) etc., are used. Selection agents such as methotrexate (MTX), micophenolic acid and G418 may be added, according to the selection marker used for transformation at gene transfection.

For gradually lowering the serum concentration to a serum-free state, the serum concentration may be lowered from about 5 to about 1%, from about 1 to about 0.1%, from about 0.1 to about 0.03%, and from about 0.03 to 0%, for instance, at every transfer of cell subculture; cells which have grown are collected.

Any culture apparatus can be used for adaptation to agitation culture in suspension, as long as it is a small culturing device equipped with a means of stirring. Such culture apparatus include spinner flasks, Techne spinner flasks, Erlenmeyer flasks and jar fermentors of various capacities.

Culture for cell adaptation to agitation culture in suspension and serum-free medium can be achieved by a known method; for example, subculture may be repeated at intervals of about 3 to about 7 days at about 30 to about 40°C and an agitation speed of 20 to about 100 rpm, with pH adjustment and aeration as necessary. Such adaptation requires about 1 to about 5 months in total. During the adaptation, the basal medium can be changed one or more time for giving to the cells adaptivity to various basal media. Thus-obtained cells have substantially lost their natural adhesiveness and serum dependency and are capable of proliferation by the agitation culture in suspension in serum free medium. In this regard, substantially no adhesiveness means that the cell shows no adhesiveness under general agitation culture conditions such as described above.

Cloning for selection of clones having high productivity can be achieved by known methods such as the limiting delution method, the colony isolation method and the microwell method [Shin Seikagaku Jikken Koza 18 (Lecture of New Biochemical Experiment 18), Saibo Baiyo Gijyutsu (Cell culture technology), edited by the Japanese Biochemical Society, pp. 12-13 (1990), Tokyo Kagaku Dojin]. Repeating the selection of high-producing clones by for example, the limiting dilution method is preferable for escalation the cell productivity. Aquiring selection agent resistance by gradually increasing the concentration of the agent is also preferable for increasing productivity, because it amplifies the structure gene encoding the desired polypeptide. A combination of these methods is also preferable.

The thus-obtained high-producing strain of biologically active polypeptide is cultured on a large-scale in serum-free medium to mass-produce the desired biologically active polypeptide. The culture apparatus used for this purpose is a known agitation culture vessel equipped with elements necessary for culture such as means of aeration, agitation, temperature control, pH control and dissolved oxygen (DO) control [Shin Seikagaku Jikken Koza 1 (Lecture of New Biochemical Experiment 1), Tanpakushitsu VI. Gosei Oyobi Hatsugen (Protein VI. Synthesis and Expression), edited by the Japanese Biochemical Society, pp. 282 and 286 (1992), Tokyo Kagaku Dojin; Shin Seikagaku Jikken Koza 18, Saibo Baiyo Gijyutsu, edited by the Japanese Biochemical Society, pp. 313-323 (1990), Tokyo Kagaku Dojin]. The air lift fermentor [J.R. Birch et al., Trends in Biotechnology, Vol. 3, p. 162 (1985)] may also be used. Available methods of culture include batch culture, feed culture (fed-batch culture) and perfusion culture, perfusion culture in suspension being advantageous for efficient culture, in which cells can be long kept viable at a high cell density by continuously or intermittently supplying fresh medium while continuously or intermittently harvesting the same amount of culture supernatant.

For perfusion culture, the culture vessel is equipped with a means for separating cells in the culture broth and the culture supernatant, a means for discharging the culture supernatant and a means for supplying fresh medium. The means for separating cells and the supernatant include filtiration, methods based on a cell precipitation such as cone type cell sedimentation column and centrifugation. More specifically, using known perfusion systems containing the culture vessel equipped such separation means and continuously accumulating the supernatant with supplying fresh medium is advantageous. Discharge of the culture supernatant and supply of fresh medium, i.e., medium replacement, is started in the period from logarithmic growth phase to stationary phase, more specifically at about 2 to about 10 days, preferably about 3 to about 7 days after culture initiation, and it is preferable that the medium replacement rate be gradually increased with the increase in cell density. Specifically, it is preferable to replace about 10 to about 100%, more preferably about 20 to about 60%, of the culture medium per day.

Serum-free media which can be used for perfusion culture in suspension for the present invention is a media for culturing animal cells containing chemically identified components without addition of unknown substance derived from serum. Usually, the serum-free medium is prepared by supplementing substituent-(s) instead of serum or its fractions. The serum-free media include the above-mentioned various basal media, with preference given to ASF-104 medium (Ajinomoto), E-RDF medium (Kyokuto Seiyaku), T medium (Nihon Seiyaku), TL-2 medium, TE medium (1:1:2 mixture of Iscove's medium, Ham's F12 medium and E-RDF medium) as supplemented with insulin, transferrin, ethanolamine, selenium, proline, polyethylene glycol etc. Selection agents such as MTX, micophenolic acid and G418 may be added as necessary to select transfected cells. Specifically, PEG-86-1 medium [Shintani et al., Applied Microbiology and Biotechnology, Vol., 27, p. 533 (1988)], PEG-TE medium (TE medium supplemented with 3.5 mg/l insulin, 6.0 mg/l transferrin, $2.5 \times 10^{-5}$ M ethanolamine, $2.5 \times 10^{-8}$ M selenious acid, 35 mg/l proline, 10 $\mu$M MTX and 1 g/l PEG) etc. are used.

The temperature of perfusion culture in suspension is normally about 30 to about 40°C, preferably around 37°C, agitation speed about 20 to about 100 rpm, preferably around 30 rpm, pH about 6 to about 8, preferably around 7, and DO about 0.5 to about 5 ppm, preferably around 1.5 ppm.

The desired biologically active polypeptide is recovered from the culture supernatant pooled in the effluent reservoir periodically or at a time after culture completion.

For separating and purifying the biologically active polypeptide produced and accumulated extracellularly by the production method of the present invention, known methods of separation and purification can be used in combination as appropriate. Such known methods of separation and purification include those based on solubility differences such as salting-out, ammonium sulfate precipitation and solvent precipitation, those based mainly on molecular weight differences such as dialysis, ultrafiltration and SDS-polyacrylamide gel electrophoresis, those based on charge differences such as ion exchange chromatography, those based on specific affinity such as affinity chromatography, those based on hydrophobicity differences such as the use of metal chelate columns, e.g., antibody columns and $Cu^{2+}$ columns, and reverse-phase high performance liquid chromatography (HPLC), and those based on isoelectric point differences such as isoelectric focusing.

The present invention makes it possible to culture biologically active polypeptide producing cells in serum-free medium by agitation culture in suspension in a long term with retaining their productivity. The production method is advantageous for industrial mass-production of a biologically active polypeptide using recombinant animal cells in view of productivity and purification efficiency. For example, the present invention allows mass-supply of biologically active polypeptide such as polypepeitde belong to human NGF family (NGF-2 etc.) by industrial production, thus contributing significantly to pharmaceutical application of the substance.

The present invention is hereinafter described in more detail by means of the following examples, but the invention is not by any means limited thereto. The animal cells obtained in Examples below have been deposited as shown in the following table.

| Animal cell | IFO (IFO No.) | NIBH (FERM No.) |
|---|---|---|
| CHO-D31-10-SFK-45 | 50403 (May 7, 1993) | BP-4297 (May 19, 1993) |
| CHO-N2-1SF | 50441 (March 23, 1994) | BP-4624 (March 31, 1994) |
| IFO: The Institute for Fermentation (Osaka); 17-85, Jusohonmachi 2-chome, Yodogawaku, Osaka, Japan NIBH: National Institute of Bioscience and Human- Technology Agency of Industrial Science and Technology; 1-3, Higashi 1-chome Tsukuba-shi, IBARAKI, Japan | | |

In the table, the date of deposit is given in parenthesis.

Example 1. Adaptation to agitation culture in suspension

A vial of frozen CHO-D31-10 cells [Iwane et al., Biochemical and Biophysical Research Communication, Vol. 171, p. 116 (1990); IFO 50217 FERM BP-2574], which produce human NGF, were rapidly thawed at 37°C, mixed with about 9 ml of serum-containing ASF104 medium (ASF104 medium supplemented with 10 $\mu$M MTX, 35 mg/l L-proline and 10% FCS) and centrifuged. After supernatant removal, the residual cells were re-suspended in about 40 ml of the same medium to a viable cell density of about $1 \times 10^5$/ml, placed in an F75 flask and cultured in an incubator at 37°C in the presence of 5% carbon dioxide for 4 days. The cells which proliferating adhered to inner wall of flask were collected via trypsin-EDTA treatment, suspended in about 10 ml of serum-containing ASF104 medium, and counted. These cells were then transferred to a 125 ml Techne spinner flask containing 50 ml of low-serum-concentration ASF104 medium (ASF104 medium supplemented with 10 $\mu$M MTX, 35 mg/l L-proline and 1% FCS) to a viable cell density of about $1.5 \times 10^5$/ml and subjected to agitation culturing at 37°C and 40 rpm. By about 1 week after culture initiation, suspended cells had proliferated to a viable cell density of about $5 \times 10^5$/ml. These suspended cells were collected, transferred to a 125 ml Techne spinner flask to a viable cell density of about $1.5 \times 10^5$/ml and again subjected to agitation culturing under the same conditions as above. The same procedure of agitation culture in suspension using a Techne spinner flask was repeated in 4 more cycles to yield cells capable of proliferation in suspension in low-serum-concentration ASF104 medium up to about $1 \times 10^6$/ml without lag phase.

Example 2. Adaptation to serum-free medium

The cells adapted to agitation culture in suspension obtained in Example 1 was transferred to a 125 ml Techne spinner flask containing 100 ml of serum-free ASF104 medium (ASF104 medium supplemented with 10 $\mu$M MTX and 35 mg/l L-proline) to a viable cell density of about $1 \times 10^5$/ml and subjected to agitation culturing at 37°C and 40 rpm. By about 8 days after culture initiation, suspended cells had proliferated to a viable cell density of about $4 \times 10^5$/ml, but no more proliferation occurred. These 8-day cultured cells were collected, transferred to a 125 ml Techne spinner flask to a viable cell density of about $1 \times 10^5$/ml and again subjected to agitation culturing under the same conditions as above. The same procedure of agitation culture in suspension using serum-free medium were repeated in 11 more cycles to yield cells capable of proliferation in suspension in serum-free ASF104 medium to a viable cell density of about $1 \times 10^6$/ml without lag phase. These cells also proliferated well in other serum-free media such as serium-free E-RDF/ASF104 (1:1 mixture) medium and other media, as well as in the serum-free ASF104 medium.

Example 3. Adaptation to serum-free agitation culture in suspension (1)

CHO-D31-10 cells described in Example 1, which produce human NGF, were transferred to a 125 ml Techne spinner flask containing 100 ml of serum-free ASF104 medium to a cell density of about $3 \times 10^5$/ml and subjected to agitation culturing at 37°C and 40 rpm. Although the cell count declined rapidly, not all subjects died, resulting in a viable cell density of about $0.5 \times 10^5$/ml about 2 weeks later. The entire amount of this liquid medium was centrifuged to separate cells, which were suspended in 30 ml of fresh serum-free ASF104 medium and cultured in a Techne spinner flask. Three days later, the medium was replaced with fresh medium and culturing was continued for 8 more days, resulting in a viable cell density of about $5 \times 10^5$/ml. These cells were collected, again transferred to a 125 ml Techne spinner flask to a viable cell density of about $1.5 \times 10^5$/ml and cultured. Such subculturing using serum-free ASF104 medium was repeated in 3 more cycles, followed by 6 cycles of subculturing using E-RDF serum-free medium and 11 cycles of subculturing using serum-free TE medium (TE medium supplemented with 3.5 mg/l insulin, 6.0 mg/l transferrin, $2.5 \times 10^{-5}$ M ethanolamine, $2.5 \times 10^{-8}$ M selenious acid (sodium selenite may use as substitute), 35 mg/l proline and 10 $\mu$M MTX), to yield cells capable of suspension proliferation in E-RDF/ASF104 (1:1 mixture) serum-free medium and TE serum-free medium to a viable cell density of about $1 \times 10^6$/ml without lag phase.

Example 4. Selection of highly productive clones by cloning

Since the human NGF productivity of the strains adapted to serum-free agitation culture in suspensin obtained in Examples 2 and 3 was about 50% lower than that of the parent cells, the cells obtained in Example 3 were cloned by the limiting dilution method to select a highly productive strain. Specifically, the culture broth was collected from the flask and centrifuged. After the cells were collected and treated with a PBS solution containing 0.03% trypsin-0.01% EDTA at 37°C for 2 minutes, they were washed with cloning medium (TE medium supplemented with 0.1% FCS, 10 $\mu$M MTX and 35 mg/l L-proline). The obtained cells were suspended in cloning medium and diluted with the same medium to a cell count of 5 per ml. This dilution was dispensed to about fifteen 96-well plates at 0.1 ml per well and cultured in an incubator at 37°C in the presence of 2.5% carbon dioxide for 18 days. After the wells in which cell grew were selected, 0.1 ml of fresh cloning medium was added. After 5 more days of culturing, the cells were transferred to 24-well plates containing 0.5 ml of serum-free TE medium per well. The amount of inoculum was increased or decreased as appropriate according to the extent of growth, with 0.1 ml as a standard. After 5 days of culturing in an incubator at 37°C in the presence of 2.5% carbon dioxide, 100 $\mu$l of the liquid medium was collected from each well and subcultured on new 24-well plates. The other portion of the liquid medium was cultured for 2 more days, after which NGF production was determined by the enzyme immunoassay method [G. Heinrich and T.E. Meyer, Biochemical Biophysical Research Communication, Vol. 155, p. 482 (1988)]. Forty-four clones of relatively high productivity were selected; 5 days later, cells were subcultured from the plates to 12-well plates, F25 flasks, F75 flasks and 125 ml Erlenmeyer flasks in that order, each containing serum-free TE medium. Twenty-four clones which proliferated well in rotary shaking culture (100 rpm) in the Erlenmeyer flask were selected and transferred to 125 ml Techne spinner flasks to a low seeding density ($0.3 \times 10^5$/ml), subjected to spinner culturing using serum-free TE medium, and one strain (SF-K45) of highest productivity was selected. The SF-K45 strain produced 10.7 mg/ml human NGF in culture using a Techne spinner flask.

Example 5. Serum-free batch culture in suspension using 2-liter jar fermentor

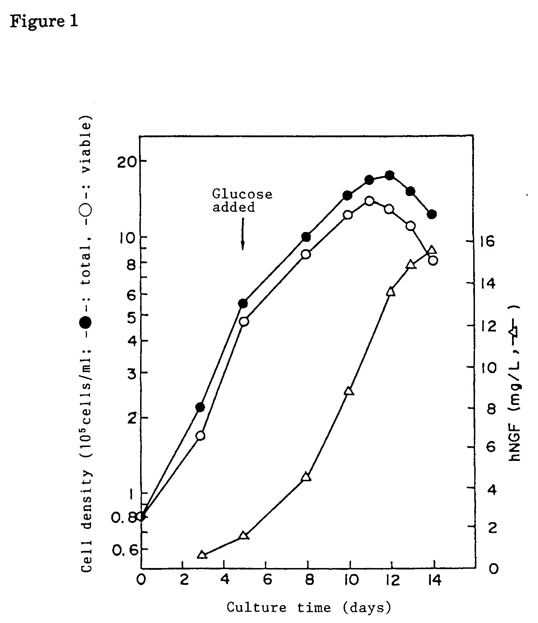

A vial of frozen cells of SF-K45 strain obtained in Example 4 were rapidly thawed at 37 °C, mixed with' about 9 ml of serum-free TE medium and centrifuged. After supernatant removal, the residual cells were suspended in about 40 ml of the same medium to a viable cell density of $1 \times 10^5$/ml, placed in an F75 flask and cultured in an incubator at 37 °C in the presence of 5% carbon dioxide for 3 days. The cells which proliferated were collected via centrifugation and transferred to a 125 ml Techne spinner flask containing 100 ml of serum-free TE medium to a viable cell density of about $1 \times 10^5$/ml and subjected to agitation culturing at 37 °C for 5 days. The cells which proliferated were transferred to a 500 ml Techne spinner flask and cultured to the late logarithmic growth phase. The cells were transferred to a 2-liter jar fermentor containing about 1 liter of serum-free PEG-TE medium (TE medium supplemented with 3.5 mg/l insulin, 6.0 mg/l transferrin, $2.5 \times 10^{-5}$ M ethanolamine, $2.5 \times 10^{-8}$ M selenious acid, 35 mg/l proline, 10 $\mu$M MTX and 1 g/l PEG) at a seeding density of about $0.8 \times 10^5$/ml, and cultured at 36 °C and an agitation speed of 30 rpm. At 5 days of culturing, 0.3% glucose was added, and culturing was continued at about pH 7.0 and dissolved oxygen concentration of about 1.5 ppm. About 15 mg/l human NGF was produced. The time course of this culturing is shown in Figure 1.

Example 6. Serum-free batch culture in suspension using 200-liter fermentor

A vial of frozen cells of SF-K45 strain obtained in Example 4 were rapidly thawed at 37 °C, mixed with about 9 ml of serum-free TE medium and centrifuged. After supernatant removal, the residual cells were suspended in about 40 ml of the same medium to a viable cell density of $1 \times 10^5$/ml, placed in an F75 flask and cultured in an incubator at 37 °C in the presence of 5% carbon dioxide for 3 days. The cells which proliferated were collected via centrifugation and transferred to a 125 ml Techne spinner flask containing 100 ml of serum-free TE medium to a viable cell density of about $1 \times 10^5$/ml and subjected to agitation culturing at 37 °C for 5 days. The cells which proliferated were transferred to a 500 ml Techne spinner flask and then to a 3-liter spinner flask and then to a 50-liter fermentor for seed culture (working volume 25 liters). Twenty liters of the culture broth, obtained by culturing in the 50-liter fermentor to the late logarithmic proliferation phase, was transferred to a 200-liter fermentor containing about 90 liters of PEG-TE serum-free medium, and culturing was initiated at 36 °C and an agitation speed of 30 rpm. At 4 days of culturing, 0.3% glucose was added, and culturing was continued at about pH 7.0 and dissolved oxygen concentration of about 1.5 ppm. About 15 mg/l human NGF was produced.

Example 7. Serum-free perfusion culture in suspension using 2-liter jar fermentor

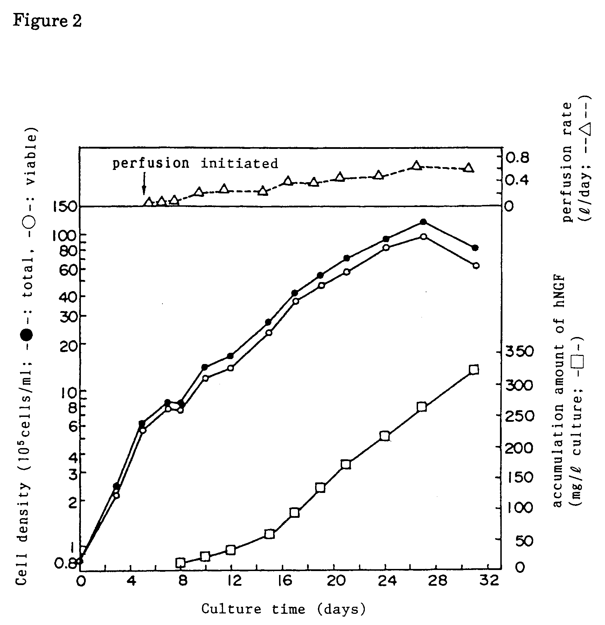

Cells which were proliferated by the same method as described in Example 5 to the late logarithmic growth phase in a 500-ml Techne spinner flask were transferred to a 2-liter jar fermentor containing about 1 liter of serum-free PEG-TE medium at a seeding density of about $0.8 \times 10^5$/ml, and culturing was initiated at 36 °C and an agitation speed of 30 rpm. At 5 days of culturing, perfusion was initiated at a medium exchange rate of about 3% (ratio of medium exchanged daily per liter of culture medium), with the medium exchange rate increased gradually to about 60% with the increase in cell density. Culturing was continued at about pH 7.0 and dissolved oxygen concentration of about 1.5 ppm. One month of culturing resulted in cell proliferation to a density of about $1 \times 10^7$/ml, equivalent to 318 mg of human NGF accumulated. The time course of this culturing is shown in Figure 2. Comparison of human NGF productivity of batch culturing and perfusion culturing is shown in Table 1. The productivity in perfusion culturing was about 20 times, about 10 times and about 2 times greater than that in batch culturing per unit reactor capacity, unit reactor capacity per culturing day and per medium consumption, respectively, demonstrating that perfusion culturing is advantageous over batch culturing.

Table 1

| Comparison of Batch Culturing and Perfusion Culturing as to hNGF Productivity | | | | | | |
|---|---|---|---|---|---|---|
| | Culture Volume (liter) | Culture Time (days) | Medium Consumption (liter) | hNGF Productivity | | |
| | | | | $(mg/\ell)^{1)}$ | $(mg/\ell/day)^{2)}$ | $(mg/\ell/medium)^{3)}$ |
| Batch culturing | 1 | 14 | 1.0 | 15 | 1.1 | 15 |
| Perfusion culturing | 1 | 31 | 10.7 | 318 | 10.3 | 30 |

1) Productivity per liter of culture volume
2) Productivity per liter of culture volume per culture day
3) Productivity per medium consumption

Example 8. Isolation of human NGF

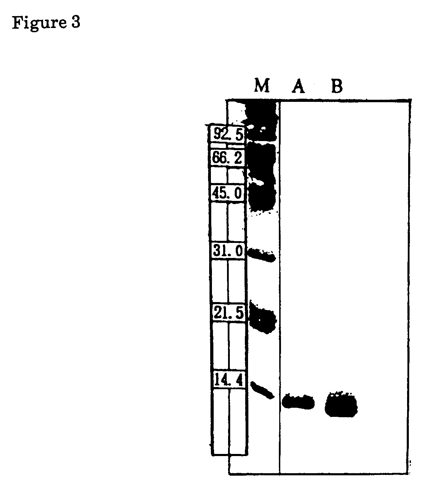

The liquid medium was centrifuged to separate the culture supernatant. After EDTA, PMSF, APMSF and CHAPS were added to respective final concentrations of 1 mM, 1 mM, 0.1 mM and 0.05%, the culture supernatant (150 liters) was concentrated using an ultrafiltration membrane (PTGC, Pericon Cassette System, Millipore Company) to yield a 6-liter concentrate. After pH adjustment to 6.0 with acetic acid, the concentrate was adsorbed to a column (5 × 43 cm) of S-Sephadex, previously equilibrated with 0.1M phosphate buffer + 1 mM EDTA + 0.05% CHAPS (pH 6.0), washed with 0.1M phosphate buffer + 1 mM EDTA + 0.05% CHAPS + 150 mM NaCl (pH 6.0) and 20 mM Tris-HCl + 1 mM EDTA + 0.1% CHAPS (pH 7.4), then eluted with 20 mM Tris-HCl + 1 mM EDTA + 0.1% CHAPS + 700 mM NaCl (pH 7.4). The fraction containing human NGF was collected and concentrated to a 1/50 to 1/100 volume using Diaflow Cell (YM-10 membrane, Amicon Corporation). The concentrate was subjected to gel filtration through a column (4.5 × 60 cm) ofToyopearl HW-50F, previously equilibrated with 20 mM Tris-HCl + 1 mM EDTA + 0.1% CHAPS + 150 mM NaCl (pH 7.4). The major eluted fraction was collected and adjusted to pH 6.5. The solution was adsorbed to a column (2.15 × 15 cm) of TSK gel SP-5PW, previously equilibrated with 20 mM phosphate buffer + 1 mM EDTA + 0.05% CHAPS + 150 mM NaCl (pH 6.5). After elution on an NaCl density gradient from 150 mM to 1M, the major eluted fraction was collected and applied to a column (2.15 × 15 cm) of TSK gel Phenyl-5PW RP. After elution on a density gradient of acetonitrile (containing 0.1% trifluoroacetic acid) from 0 to 80%, the major eluted fraction was collected and lyophilized (yield 419 mg). Chromatography was again conducted, using the same conditions as above, to yield a 155 mg purified preparation of human NGF. This preparation was subjected to protein chemical analysis. Tables 2, 3 and 4 show the results on amino acid composition, those on N-terminal amino acid sequence and those on C-terminal amino acid respectively. The results of SDS-PAGE (gel for concentration 6%, gel for separation 15%, reduction conditions 50 mM DTT, 100 °C, 2 minutes, silver staining) are shown in Figure 3. Using PC-12 cells, the bioactivity of the purifiedpreparation was found to be almost equal to that of mouse NGF.

Table 2

| Amino Acid Composition Analysis | | |
|---|---|---|
| Amino Acid | Experimental Value (residues/mole) | Theoretical Value [3] |
| Asx | 12.97 | 13 |
| Thr | 9.30 | 10 |
| Ser | 9.50 | 11 |
| Glx | 6.50 | 6 |
| Pro | 2.93 | 3 |
| Gly | 7.00 | 7 |
| Ala | 6.74 | 7 |
| Cys[1] | | 6 |
| Val | 12.37 | 13 |
| Met | 1.81 | 2 |
| Ile | 5.79 | 6 |
| Leu | 3.08 | 3 |
| Tyr | 1.92 | 2 |
| Phe | 6.13 | 7 |
| His | 3.94 | 4 |
| Lys | 9.00 | 9 |
| Arg | 7.79 | 8 |
| Trp[2] | 3.06 | 3 |
| Acid hydrolysis (values obtained after 24 hours of hydrolysis with 6N HCl-1% phenol at 110°C) | | |

1) Not examined

2) Determined by Edelhoch's method, using about 20 $\mu$g sample.

3) Theoretical value was estimated from nucleotide sequence of NGF

Table 3

| N-terminal Amino Acid Analysis | | |
|---|---|---|
| Residue No. | Detected Amino Acid (pmole) | Amino Acid Estimated from Nucleic acid Sequence of NGF |
| 1 | Ser (298) | Ser |
| 2 | Ser (289) | Ser |
| 3 | Ser (222) | Ser |
| 4 | His (37) | His |
| 5 | Pro (288) | Pro |
| 6 | Ile (315) | Ile |
| 7 | Phe (352) | Phe |
| 8 | His (29) | His |
| 9 | Arg (51) | Arg |
| 10 | Gly (292) | Gly |
| 11 | Glu (114) | Glu |
| 12 | Phe (243) | Phe |
| 13 | Ser (79) | Ser |
| 14 | Val (192) | Val |
| 15 | X | Cys |
| 16 | Asp (80) | Asp |
| 17 | Ser (-) | Ser |
| 18 | Val (171) | Val |
| 19 | X | Ser |
| 20 | Val (155) | Val |
| Analyzed with a 1 nmol sample, using a gas-phase protein sequencer model 477A (produced by Biosystem Company). X: Not examined | | |

Table 4  C-terminal Amino Acid Analysis

| NGF | C-terminal Amino Acid | Recovery (%) |
|---|---|---|
| | Ala | 25.3 |

15 nmole sample used.

Example 9. Adaptation to serum-free agitation culture in suspension (2)

A vial of frozen CHO-N2-1 cells which produce human NGF-2 (EPO 386752) were rapidly thawed at 37°C, mixed with about 9 ml of serum containing TE medium (TE medium supplemented with 2μM MTX, 35 mg/l L-proline and 10%FCS) and centrifuged. After supernatant removal, the residual cells were re-suspended in about 50 ml of the same medium to a viable cell density of about 1 x $10^5$/ml, placed in an F150 flask and cultured in an incubator at 37°C in the presence of 5% carbon dioxide for 4 days. The cells which proliferated adhered to the inner wall of flask were collected via trypsin-EDTA treatment. These cells were transferred to a 125 ml Techne spinner flask containing 50 ml of serum-free TE medium (TE medium supplemented 3.5 mg/l insulin, 6.0 mg/l transferrin, 2.5 x $10^{-5}$ M ethanolamine, 2.5 x $10^{-8}$ M selenious acid and 2μM MTX) at a viable cell density of about 1 × $10^5$/ml and subjected to agitation culturing at 37°C and 30 rpm. Although the cell number declined about half, culturing was continued, resulting in a viable cell density of about 4 × $10^5$/ml about 9 days later. The entire amount of this culture broth was centrifuged to separate cells, which were suspended in about 100 ml of fresh serum-free TE medium to a viable cell density of about 1 x $10^5$/ml and cultured in a Techne spinner flask for 5 days (37°C, 30 rpm), resulting in a viable cell density of about 5 × $10^5$/ml. These cells were collected, again transferred to a 125 ml Techne spinner flask to a viable cell density of about 1 × $10^5$/ml and cultured at 37°C, 50 rpm for 6 days. Such subculturing using serum-free TE medium was repeated in 7 more cycles, to yield cells capable of proliferation in serum-free TE medium in suspension to a viable cell density of about 1 × $10^6$/ml without lag phase. Thus obtained adaptation cells were named CHO-N2-1SF.

Example 10. Adaptation to serum-free medium

The CHO-N2-1SF cells obtained in Example 9 were transferred to a 125 ml Techne spinner flask containing 100 ml of serum-free PEG-TE medium (TE medium supplemented with 1 g/l PEG) at a viable cell density of aboutd 1 x $10^5$/ml and subjected to agitation culturing at 37°C and 50 rpm. At 6 days of culturing 0.3% glucose was added, and culturing was continued at about pH 7.0 for 15 more days. As a result, about 2 mg/l of human NGF-2 was produced. The NGF-2 was determined by the known enzyme immunoassay (EIA) [Biochem. Biophys. Res. Commun., Vol. 194 No. 3, p 1500 (1993)].

**Claims**

1. A method for producing a biologically active polypeptide for an extended period of time which comprises:
   (a) adapting an adhesion-dependent animal cell capable of producing said polypeptide to be an adhesion-independent cell;
   (b) adapting said cell to grow in a serum-free medium;
   (c) culturing said cells in suspension culture in serum-free medium under conditions suitable for expression of said polypeptide; and
   (d) recovering said polypeptide.

2. The method of claim l, wherein the extended period of time is more than 20 days.

3. The method of claim l, wherein the extended period of time is more than 40 days.

4. The method of claim l, wherein the extended period of time is more than 60 days.

5. The method of claim l, wherein said adhesion-dependent animal cells are Chinese hamster ovary cells.

6. The method of claim l, wherein said biologically active polypeptide is a peptide belonging to the nerve growth factor family.

7. The method of claim l, wherein said polypeptide is a polypeptide belonging to the human nerve growth factor family.

8. The method of claim 6, wherein said polypeptide is NGF.

9. The method of claim 6, wherein said peptide is NGF-2.

**10.** The method of claim I, wherein said suspension culture is an agitation culture in suspension or a perfusion culture.

**11.** Chinese hamster ovary cells which produce a polypeptide belonging to the nerve growth factor family and which are capable of proliferation in suspension in serum-free medium for an extended period of time.

**12.** The cells of claim 11, wherein said polypeptide is NGF.

**13.** The cells of claim 11, wherein said polypeptide is NGF-2.

Figure 1

Figure 2

Figure 3

## DOCUMENTS CONSIDERED TO BE RELEVANT

EP 94107586.3

| Category | Citation of document with indication, where appropriate, of relevant passages | Relevant to claim | CLASSIFICATION OF THE APPLICATION (Int. Cl.5) |
|---|---|---|---|
| X | EP - A - 0 499 993 (TAKEDA CHEMICAL INDUSTRIES) * Claims 1,2,5-8 * -- | 1, 5-9, 11-13 | C 12 N 15/12 C 12 N 15/85 C 12 N 15/18 C 12 P 21/00 |
| X | WO - A - 91/07 497 (KABIGEN) * Claims 1,6-14 * -- | 1, 5-8, 11,12 | C 12 N 5/06 C 12 N 5/16 |
| X,D | BIOCHEMICAL AND BIOPHYSICAL RESEARCH COMMUNICATIONS, vol. 171, no. 1, August 1990 M.IWANE et al."Production, Purification and Characterization of Biologically Active Recombinant Human Nerve Growth Factor" pages 116-122 * Totality * -- | 1, 5-8 | |
| X | CHEMICAL ABSTRACTS, vol. 117, no. 3, July 20, 1992, Columbus, Ohio, USA C.SORANZO et al."Synthesis and expression of recombinant human nerve growth factor in a mammalian cell line." page 164, no. 21 149e; & Growth Factors Vasc. Nerv.Syst.Int.Symp. Biotechnol.Growth Factors; Vasc.Nerv.Syst. 1991 (Pub.1992), 71-9. ---- | 1, 5-8 | TECHNICAL FIELDS SEARCHED (Int. Cl.5) C 12 N C 12 P |

The present search report has been drawn up for all claims

| Place of search | Date of completion of the search | Examiner |
|---|---|---|
| VIENNA | 19-08-1994 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document